# EUROPEAN PATENT SPECIFICATION

(11) **EP 0 842 166 B1**
(45) Date of publication and mention of the grant of the patent: **08.11.2000**
(21) Application number: 96926210.4
(22) Date of filing: 31.07.1996
(51) Int. Cl.: C07D 495/08

(54) **PROCESS FOR SYNTHESIZING CARBAPENEM SIDE CHAIN INTERMEDIATES**
VERFAHREN ZUR HERSTELLUNG VON ZWISCHENPRODUKTEN VON CARBAPENEM-SEITENKETTEN
PROCEDE DE SYNTHESE DE PRODUITS INTERMEDIAIRES DE CHAINES LATERALES DE CARBAPENEM

(30) Priority: 04.08.1995 US 1891; 13.02.1996 GB 9602881
(43) Date of publication of application: 20.05.1998
(73) Proprietor: MERCK & CO. INC., Rahway New Jersey 07065-0900 (US)
(72) Inventor: BRANDS, Karel, M., J., Rahway, NJ 07065 (US)
(74) Representative: Hiscock, Ian James
(86) International application number: US9612552
(87) International publication number: WO9706154

(56) References cited:
- EP-A- 0 551 993
- US-A- 4 321 383
- H. MATSUMURA ET AL.: HETEROCYCLES, vol. 41, no. 1, 1995, pages 147-159, XP002076865
- HETEROCYCLES, 01 January 1995, Vol. 41, MATSUMURA et al., "An Efficient Synthesis of (2S,4S)-2-Substituted 4-Mercaptopyrrolidine Derivatives", pages 147-159.

## Description

### BACKGROUND OF THE INVENTION

The present invention relates to the synthesis of carbapenem side chains, and in particular, to side chains or portions thereof containing a pyrrolidine group, which is bonded to the carbapenem nucleus through a thioether linkage. Typically, the pyrrolidine is a portion of the side chain, and is substituted at the two position with any of a variety of substituents.

Conventionally, these intermediate compounds are prepared from a 4-hydroxyproline derivative of the formula: 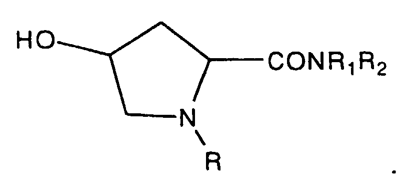 Such synthetic schemes typically require the extensive use of protecting groups.

Similarly, a method of converting trans-4-hydroxy-L-proline to a thiolactone of the formula: 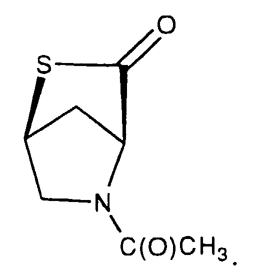 has been described. However, this thiolactone is unsuitably protected for large scale synthesis of carbapenem antibiotics.

EP 551 993 A1 published on July 21, 1993 relates to a synthesis which utilizes active esterifying agents and base, followed by treatment with hydrogen sulfide and base.

The present invention is an improvement over these other processes, utilizing an activating agent, namely, diphenylphosphinic chloride, which surprisingly improves the results which are achieved when commercial quantities are synthesized.

### SUMMARY OF THE INVENTION

A process for synthesizing a compound of the formula I: 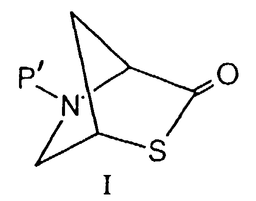 is described wherein P' is a protecting group selected from t-butoxycarbonyl, p-nitrobenzyloxycarbonyl, benzyloxycarbonyl and allyloxycarbonyl,
comprising
(a) reacting a compound of formula II: 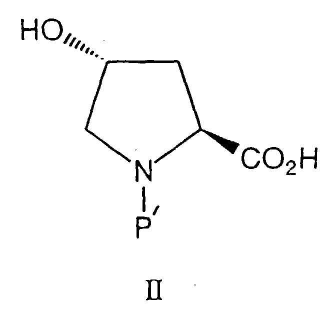 wherein P' is as previously defined with diphenylphosphinic chloride to produce a compound of formula III: 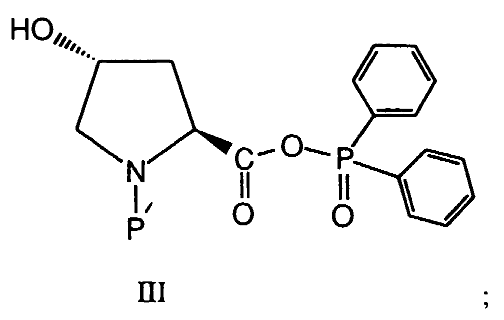
(b) reacting compound III with methanesulfonyl chloride to produce a compound of formula IV: 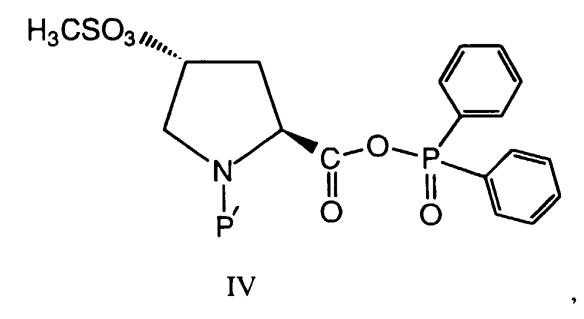 and
(c) combining compound IV with Na₂S in water to produce a compound of formula I.

Another aspect of the process described herein relates to a process for producing a compound of the formula V: 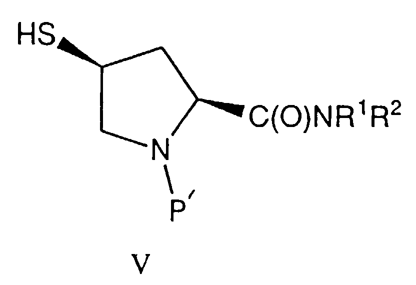 wherein P' is a protecting group selected from t-butoxycarbonyl, p-nitrobenzyloxycarbonyl, benzyloxycarbonyl and allyloxycarbonyl;
R¹ and R² are independently selected from hydrogen, aryl and heteroaryl, said aryl and heteroaryl groups being unsubstituted or substituted with from 1-3 groups selected from the group consisting of: C₁₋₄ alkyl, C₁₋₄ alkoxy, C₁₋₄ alkylthio, halo, hydroxy, CO₂H, CO₂C₁₋₄ alkyl, NH₂, NHC₁₋₄ alkyl, N(C₁₋₄ alkyl)₂, SO₃H, CN, SO₂NH₂, SO₂C₁₋₄ alkyl, aryl and heteroaryl; comprising:
(a) reacting a compound of the formula II: 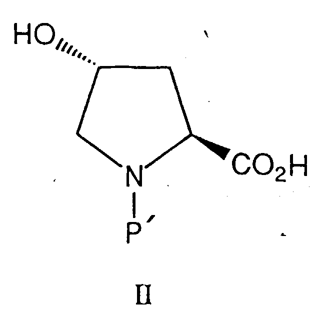 wherein P' is as previously defined with diphenylphosphinic chloride to produce a compound of the formula III: 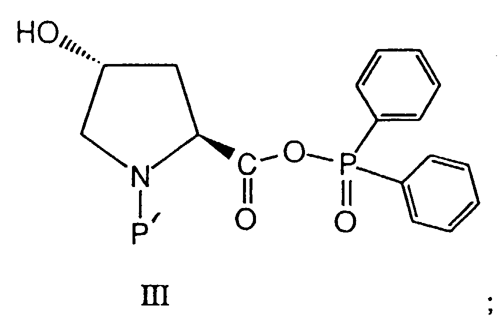
(b) reacting compound III with methanesulfonyl chloride to produce a compound of formula IV: 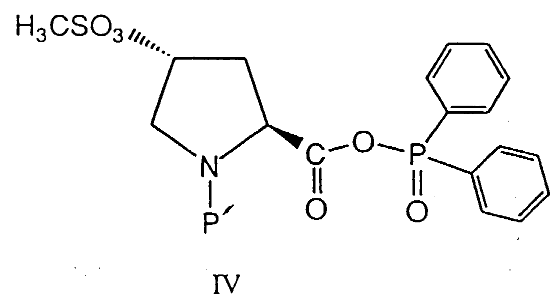
(c) combining compound IV with Na₂S in water to produce a compound of formula I: 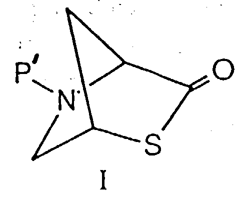
and (d) reacting compound I with NHR¹R² wherein R¹ and R² are as previously defined to produce a compound of formula V.

### DETAILED DESCRIPTION OF THE INVENTION

The following definitions apply to the terms used herein unless otherwise defined.

Alkyl and the alkyl portions of substituent groups include monovalent hydrocarbon chains containing from 1-4 carbon atoms which are straight or branched as appropriate.

Aryl refers to 6-10 membered mono- and bicyclic ring systems, containing carbon atoms with alternating (resonating) double bonds. Preferred aryl groups are phenyl and naphthyl.

Heteroaryl refers to aromatic 5-10 membered mono- and bicyclic ring systems, containing from 1-4 heteroatoms, O, S or N. Preferred nitrogen containing monocyclic heteroaryl groups include pyridyl, pyrimidinyl, pyrazinyl, pyrrolyl, imidazolyl, thiazolyl, oxazolyl and 1, 2, 4-triazolyl. Preferred heteroaryl groups containing oxygen as the only heterotom include furanyl. Preferred heteroaryl groups containing sulfur as the only heterotom include thienyl.

Preferred bicyclic heteroaryl groups include benzthiazolyl, benzimidazolyl, quinolinyl and isoquinolinyl, indolyl and isoindolyl.

When substituted, the aryl and heteroaryl groups may be substituted with 1-3 groups selected from the group consisting of: C₁₋₄ alkyl, C₁₋₄ alkoxy, C₁₋₄ alkylthio, halo, hydroxy, CO₂H, CO₂C₁₋₄ alkyl, NH₂, NHC₁₋₄ alkyl, N(C₁₋₄ alkyl)₂, NHC(O)C₁₋₄ alkyl, SO₃H, CN, SO₂NH₂, SO₂C₁₋₄ alkyl, aryl and heteroaryl.

When necessary, the substituents which are optionally present on aryl and heteroaryl can be in protected form. Examples of suitable protecting groups are: t-butylmethoxyphenylsilyl, t-butoxydiphenylsilyl, trimethylsilyl, triethylsilyl, o-nitrobenzyloxycarbonyl, p-nitrobenzyloxycarbonyl, benzyloxycarbonyl, t-butyloxycarbonyl, 2,2,2-trichloroethyloxycarbonyl benzhydryl, o-nitrobenzyl, p-nitrobenzyl, 2-naphthylmethyl, allyl, 2-chloroallyl, benzyl, 2,2,2-trichloroethyl, trimethylsilyl, t-butyldimethylsilyl, t-butyldiphenylsilyl, 2-(trimethylsilyl)ethyl, phenacyl, p-methoxybenzyl, acetonyl, p-methoxyphenyl, 4-pyridylmethyl, t-butyl and allyloxycarbonyl. Preferred hydroxyl protecting groups are trimethylsilyl and triethylsilyl. Preferred carboxyl protecting groups are p-nitrobenzyl and allyl.

Many other suitable hydroxyl and carboxyl protecting groups are known in the art. See, e.g., Greene, T. W., et al. Protective Groups in Organic Synthesis, John Wiley & Sons, Inc., 1991.

P represents a protecting group on the proline nitrogen atom. Values of P are selected from t-butoxycarbonyl (t-BOC), p-nitrobenzyloxycarbonyl, benzyloxycarbonyl and allyloxycarbonyl. The most preferred P groups are t-butoxycarbonyl and p-nitrobenzyloxycarbonyl (PNZ).

Compound II used herein as a starting material is N protected trans-4-hydroxy-L-proline. The 2-carboxyl group is activated using the compound diphenylphosphinic chloride, which is reacted with compound II in a solvent in the presence of excess base. Solvents which are useful herein include dichloromethane, acetonitrile, toluene and tetrahydrofuran, or mixtures thereof. Bases which are useful for this reaction include trialkylamines. Preferred trialkylamines include diisopropylethylamine (DIPEA) and triethylamine.

Typically an amount of diphenylphosphinic chloride which is about equimolar to the starting compound can be used. The reaction between compound II and diphenylphosphinic chloride is typically run at reduced temperature, below about 0°C to as low as about -40°C. Preferably, the reaction temperature is maintained at about -10°C.

Compound III, with the diphenylphosphinyloxycarbonyl group at position two, is reacted with methanesulfonyl chloride (MsCl) in the same pot to produce compound IV. This reaction is conducted in a solvent, in the presence of a slight molar excess of pyridine, collidine, lutidine and the like, using a slight molar excess of MsCl. This mesylation reaction may be conducted over about 1-4 hours, at a reduced temperature, e.g., about 0°C to as low as about -40°C. Preferably, the reaction temperature is maintained at about -10°C.

Compound IV is thereafter combined with sodium sulfide and water to form the thiolactone I. Essentially the reaction can be conducted at about -10°C to about room temperature. Preferably the sodium sulfide and water are added quickly, and the reaction is aged for several hours at ambient temperature.

After conversion of compound IV to compound I is complete, the latter is combined in the same pot with ammonia or a primary or secondary amine to form compounds of formula V. At this point, other solvents, such as isopropanol, ethanol, n-propanol, toluene, acetonitrile, ethyl acetate and others are added to improve crystallization of compound V, and thus facilitate its isolation. Also, addition of a trialkyl or triaryl phosphine, e.g., tri-n-butylphosphine, at this stage may be useful in reducing the formation of disulfides corresponding to compound V.

Most primary and secondary amines HNR¹R² wherein R¹ and/or R² represent H, aryl or heteroaryl react with compound I upon slight heating. Generally, the reaction proceeds from about RT to about 100°C over a few minutes to several hours.

The invention described herein can be conducted in essentially a single reaction vessel, thus allowing for economical production of compounds V from compound II.

The invention is further illustrated with the following nonlimiting examples.

### EXAMPLE ONE

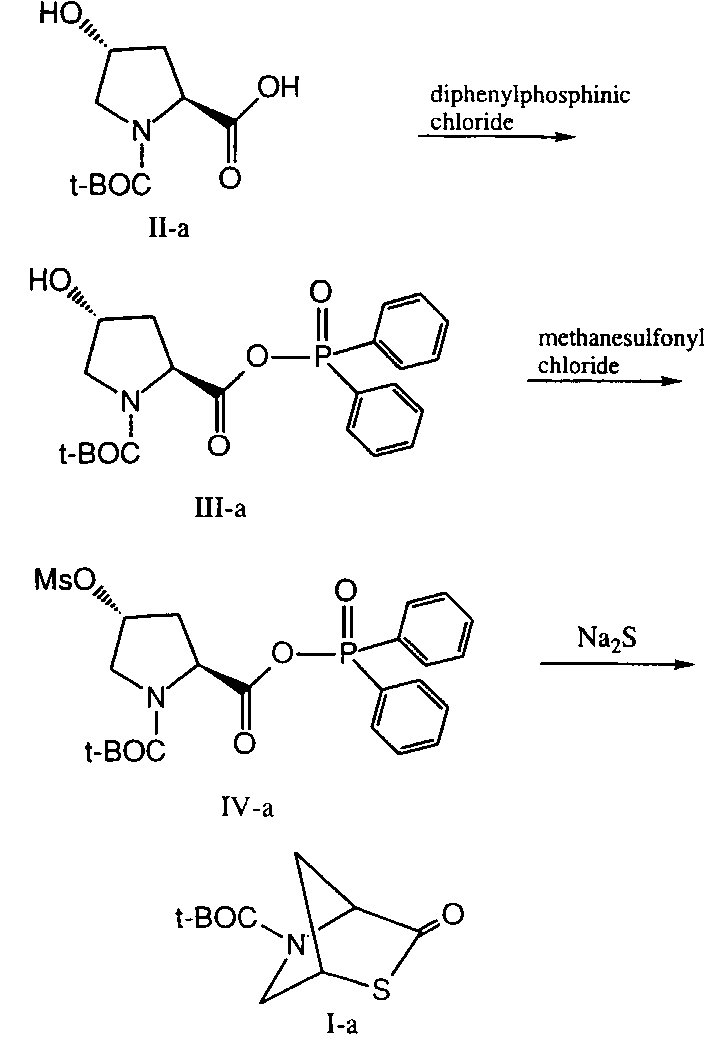

A. Synthesis of trans-N-t-butoxycarbonyl-2-diphenylphosphinyloxycarbonyl-4-hydroxy-L-proline

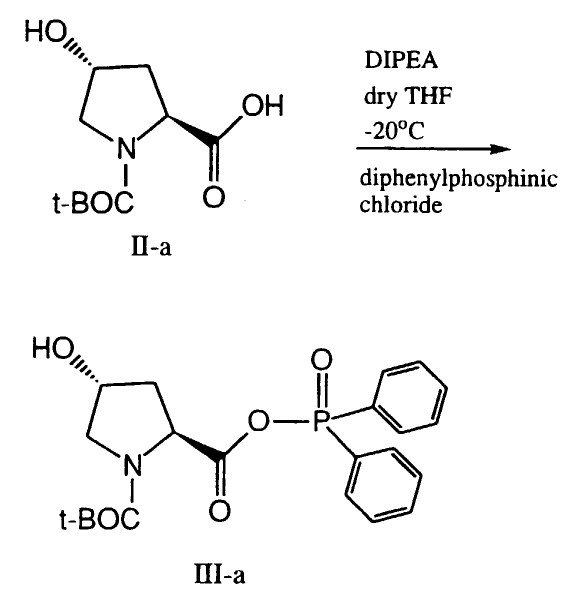

A solution of compound II-a (35.0 g, 151 mmol.) and DIPEA (60 mL, 344 mmol) in dry THF (1.0 L) was combined over 20 min with a solution of diphenylphosphinic chloride (37.5 g, 155 mmol) in THF (50 mL) at -20°C. The reaction mixture was stirred at -20°C for 90 minutes to produce compound III-a, which can be isolated and characterized or used in the next part without isolation.

B. Synthesis of trans-N-t-butoxycarbonyl-2-diphenylphosphinyloxycarbonyl-4-methanesulfonyloxy-L-proline

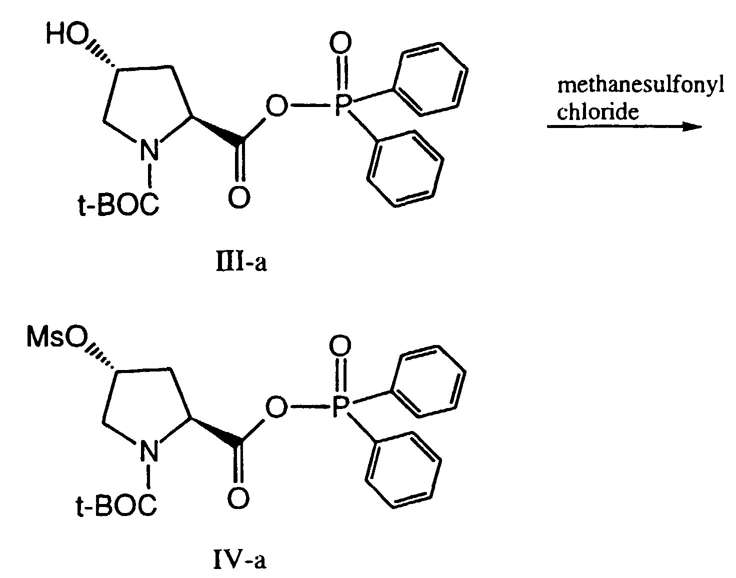

Without isolation and characterization, after stirring the reaction mixture from part A for 90 minutes at -20°C, pyridine (13.0 mL, 161 mmol) was added followed by a solution of methanesulfonyl chloride (19.8 g, 171 mmol) in THF (50 mL) over 15 minutes. The reaction mixture was stirred at -20°C for 2 hours and allowed to warm to -5°C over an additional 30 minutes producing compound IV-a. The methanesulfonyl substituted compound can be isolated and characterized, or used in the next reaction without isolation and characterization.

C. Synthesis of N-t-butoxycarbonyl-2-thia-5-azabicyclo[2.2.1]heptan-3-one

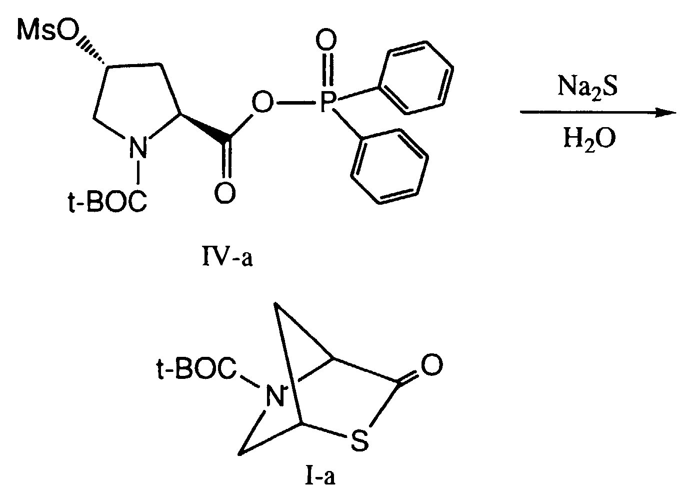

After allowing the reaction from part B to warm to -5°C, a solution of Na₂S•H₂O (45.0 g, 187 mmol) in H₂O (60 mL) was added in one portion producing a biphasic reaction mixture. The biphasic mixture was allowed to warm to room temperature and was stirred for 6 hrs. The resulting suspension was then partitioned between toluene and water.

The organic layer was washed with HCl (2.0 M), NaHCO₃ (1.0 M) and brine, dried over MgSO₄ and concentrated *in vacuo* to produce an oily residue. Crystallization of the oily residue from ether/ethyl acetate provided the title compound (29.4 g, 88 mmol).

mp 91°C; [α]_{D} = -88.0° (c = 1.01; CHCl₃). ¹H-NMR (CD₂Cl₂, -20°C; 400MHz) δ 1.42 (s,³H), 2.07 (dt, *J* 2.5, *J* 11.3, 1H), 2.13 (m, *J* 11.3, 1H), 3.48 and 3.53 (m, *J* 1.1, *J* 10.2, 1H), 3.74, (m, *J* 2.8, *J* 10.1, 1H), 4.11 (m, 1H), 4.42 and 4.53 (m, *J* 0.9, 1H); ¹³C-NMR (CD₂Cl₂, -20°C; 100 MHz) δ 29.9/30.0(q), 43.3/43.9 (1), 49.9/50.6 (d), 54.3/54.7 (t), 65.3/66.1 (d), 82.5/82.6 (s), 155.5/155.7 (s), 201.1/201.6 (s).

### EXAMPLE TWO

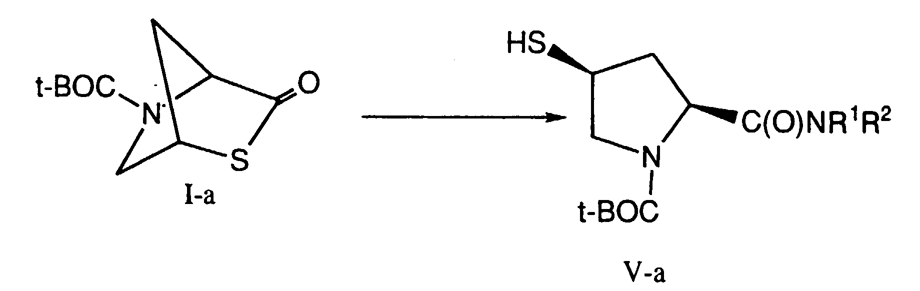

Thiolactone I-a from Example One without isolation, can be combined with the amine shown below in column one to produce the cis N-protected 4-thiol substituted proline derivative shown below in column two.

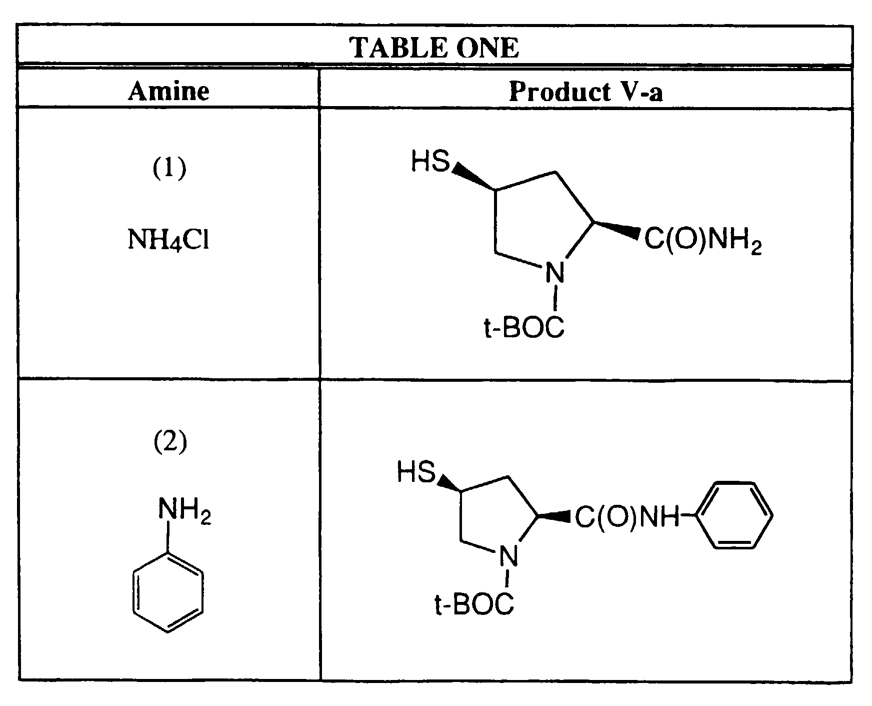

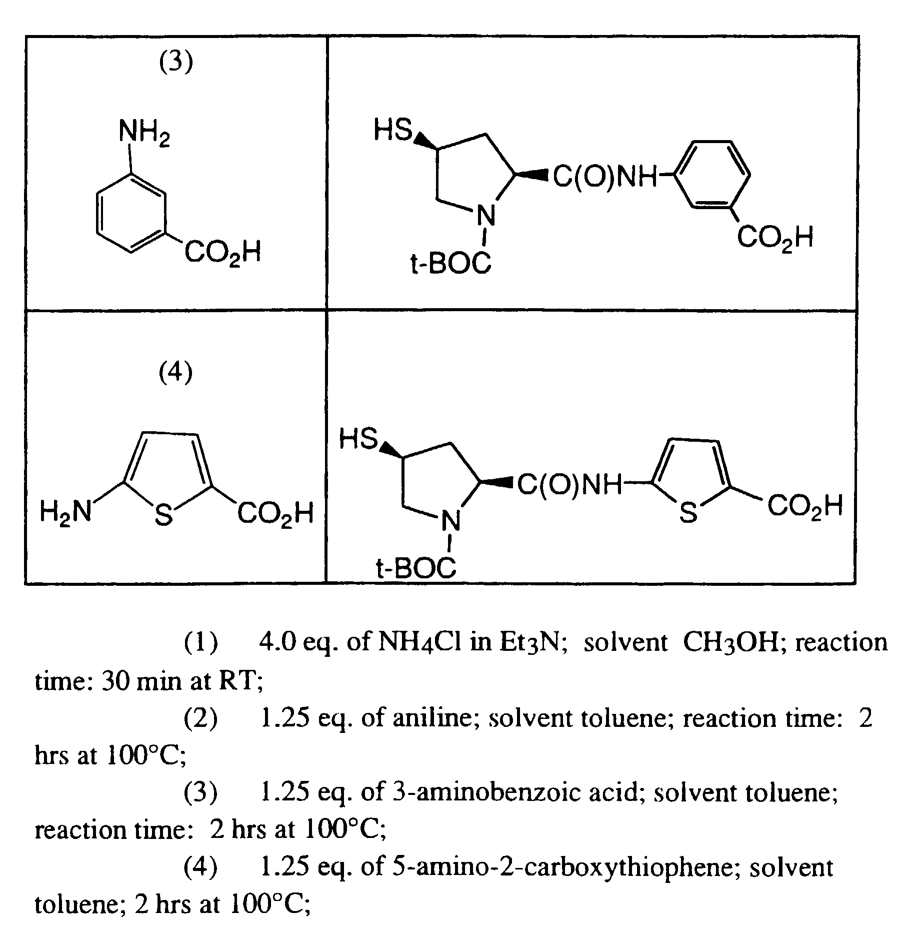

### EXAMPLE THREE

Using the procedures set forth in Example One, Part A, the compounds of column one are reacted with diphenylphosphinic chloride to produce the compounds in column two.

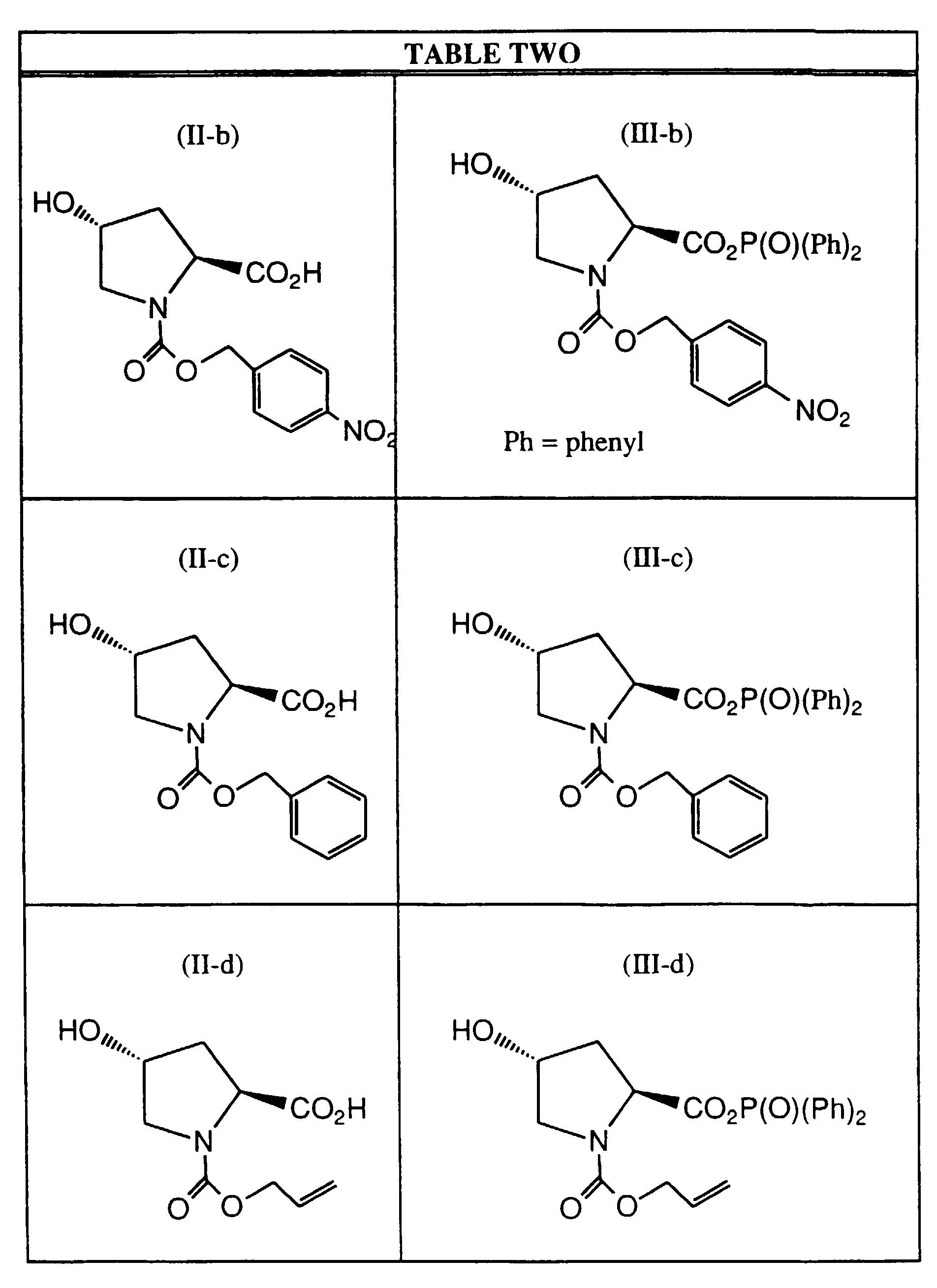

### EXAMPLE FOUR

Using the procedures set forth in Example One, Part B, the compounds of column one are reacted with methanesulfonyl chloride to produce the compounds in column two.

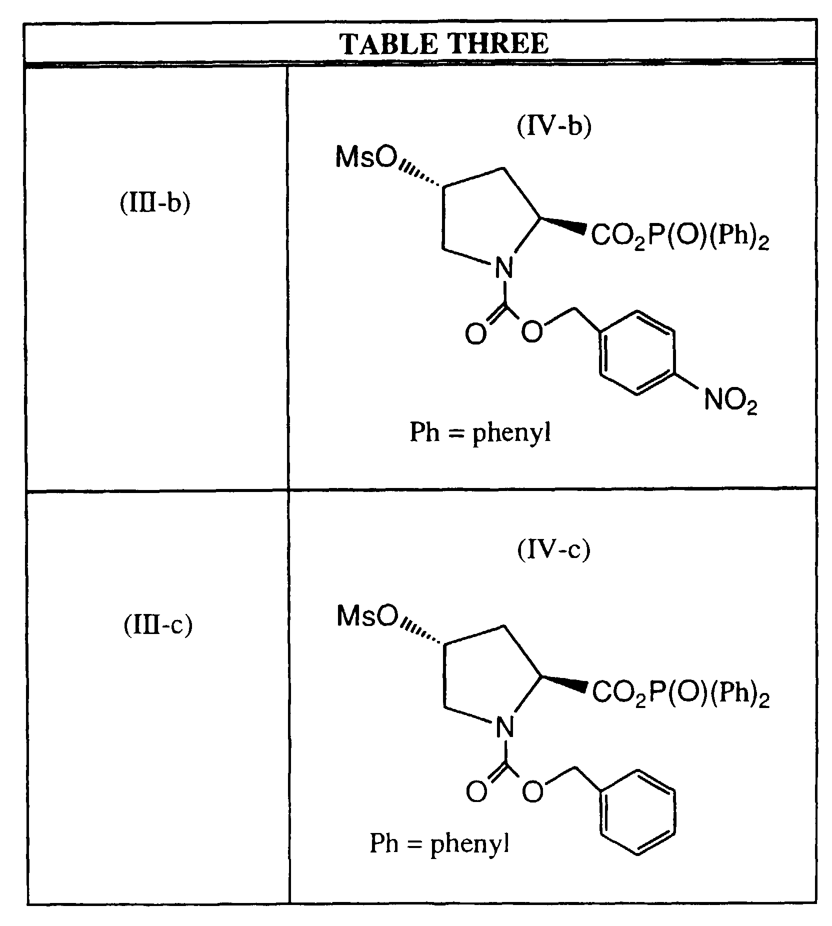

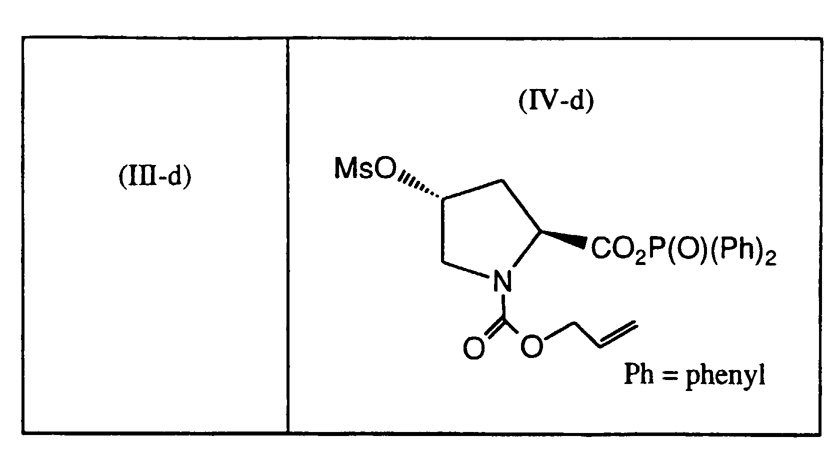

### EXAMPLE FIVE

Using the procedures set forth in Example One, Part C, the compounds of column one are reacted with Na₂S in water to produce the compounds in column two.

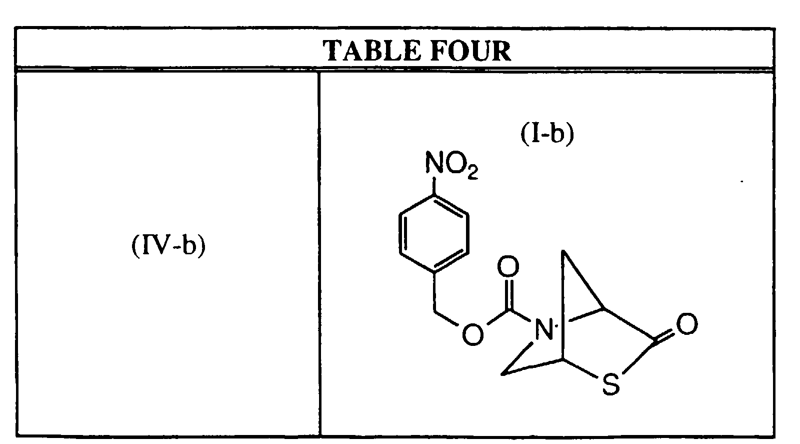

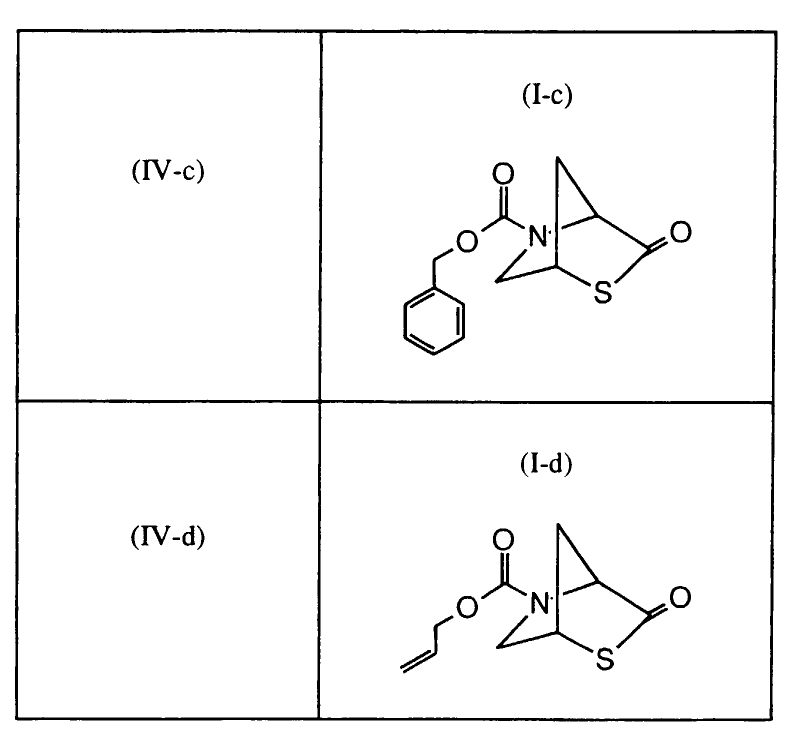

### EXAMPLE SIX

Using the procedures set forth in Example Two, the compounds of column one are reacted with the amine in column two to produce the compounds in column three.

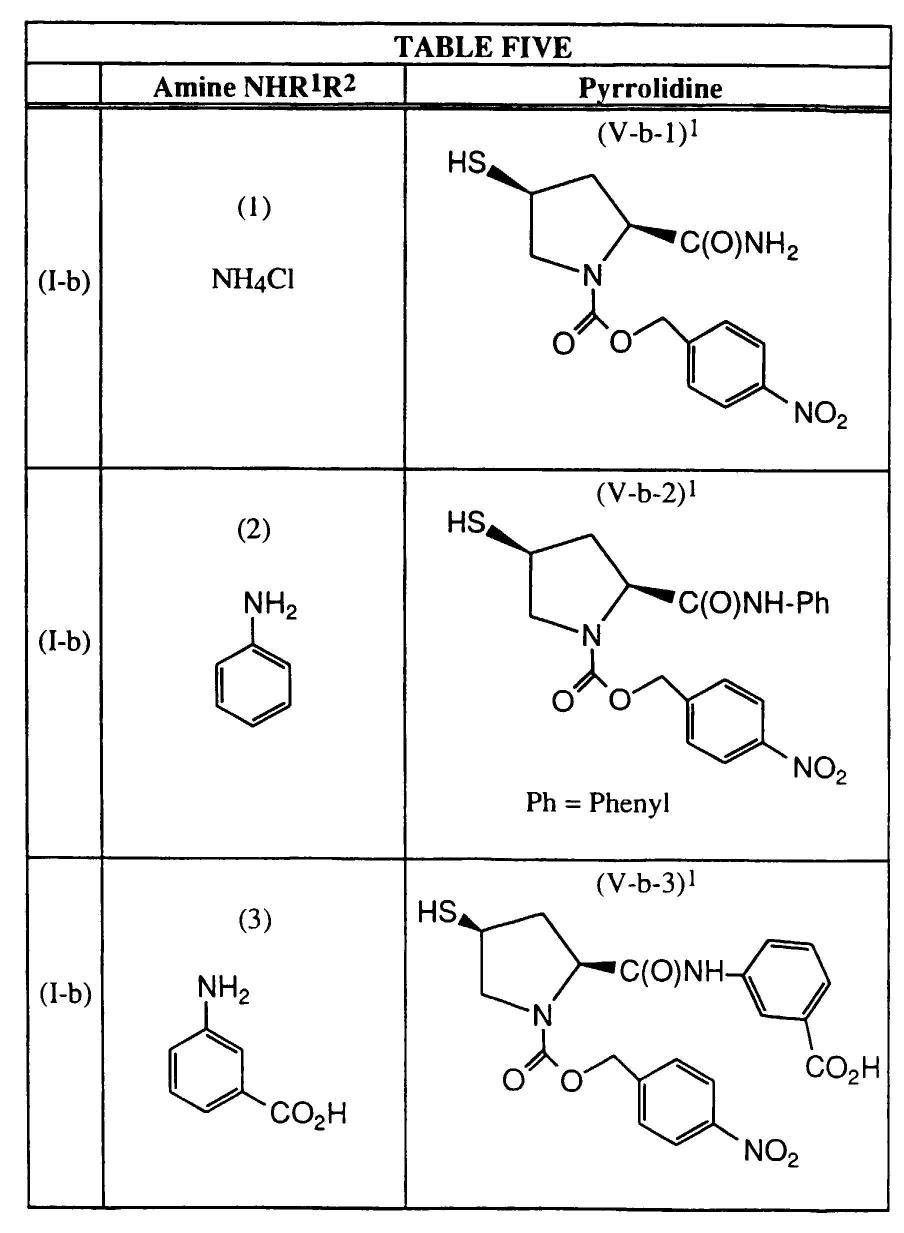

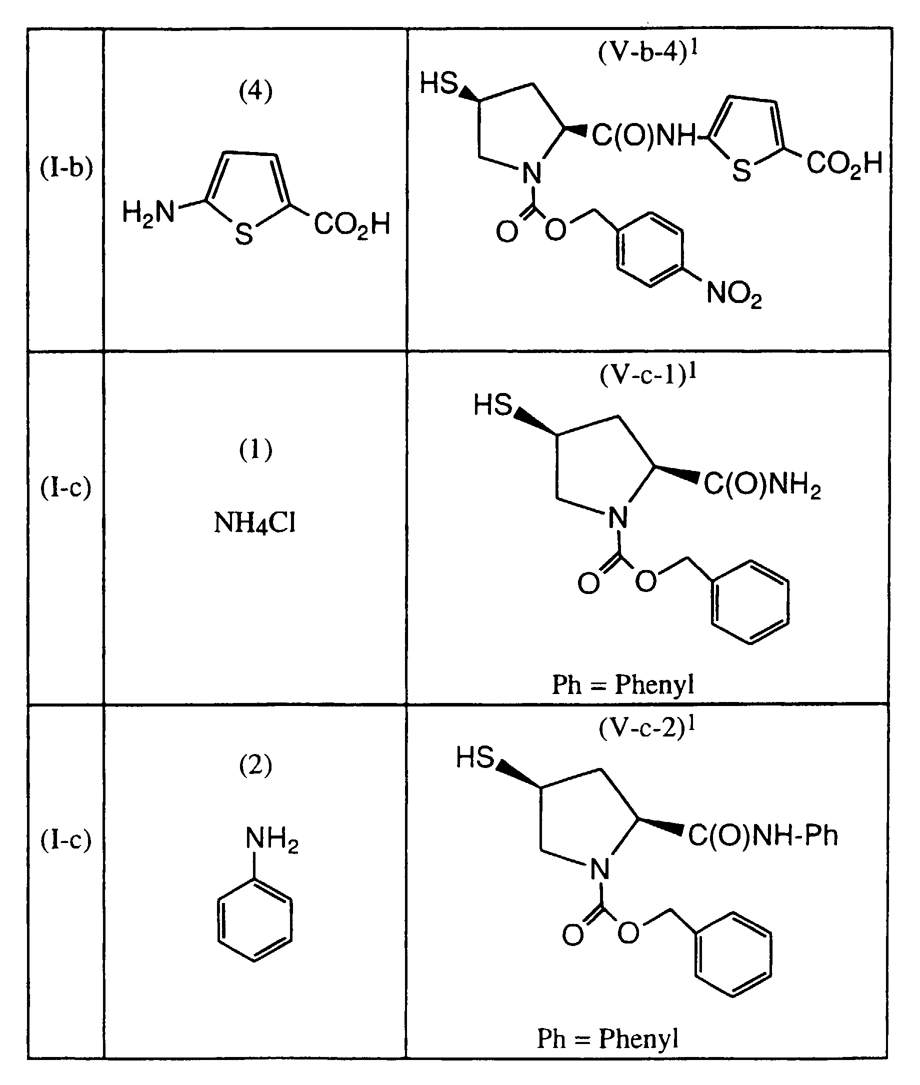

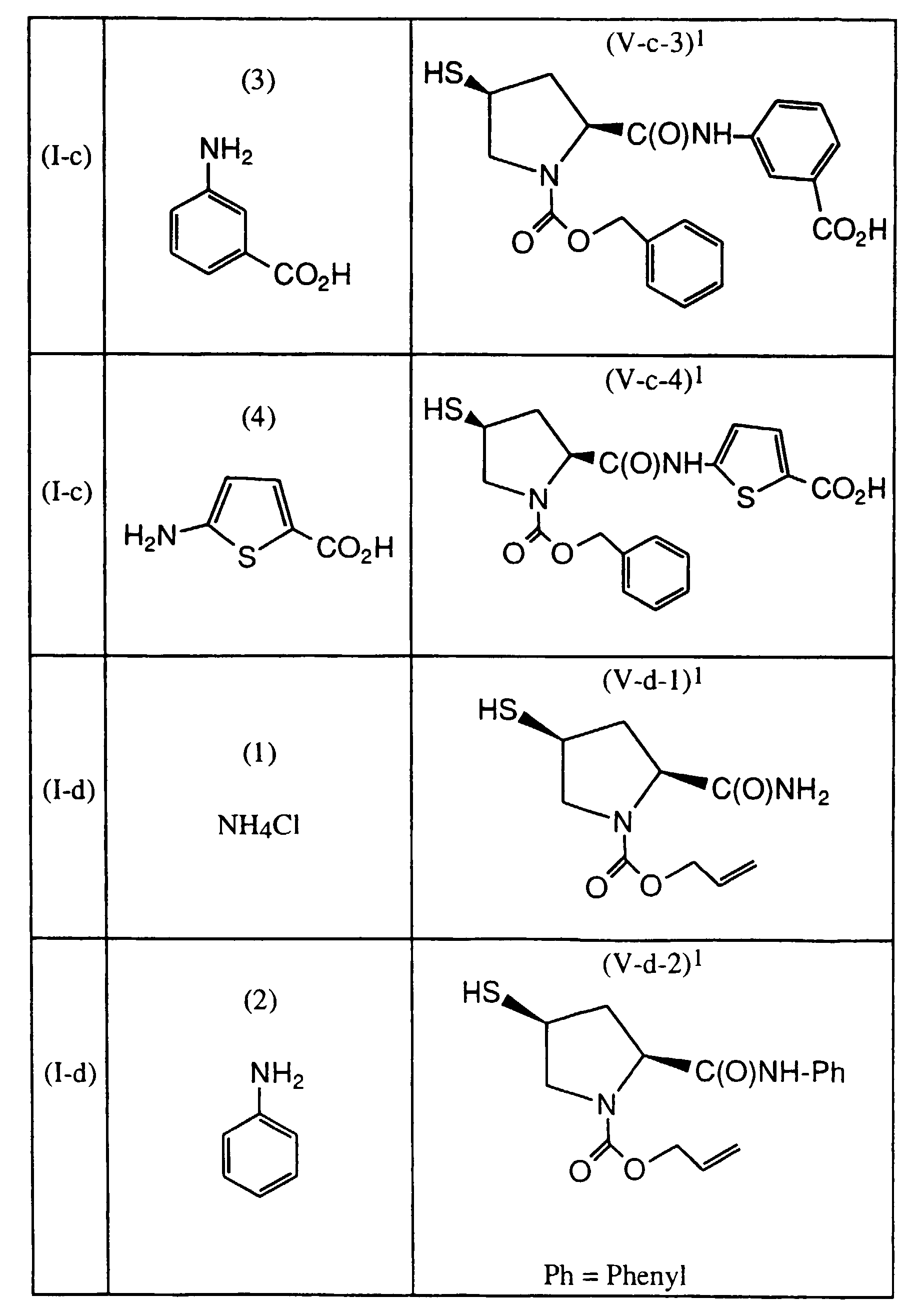

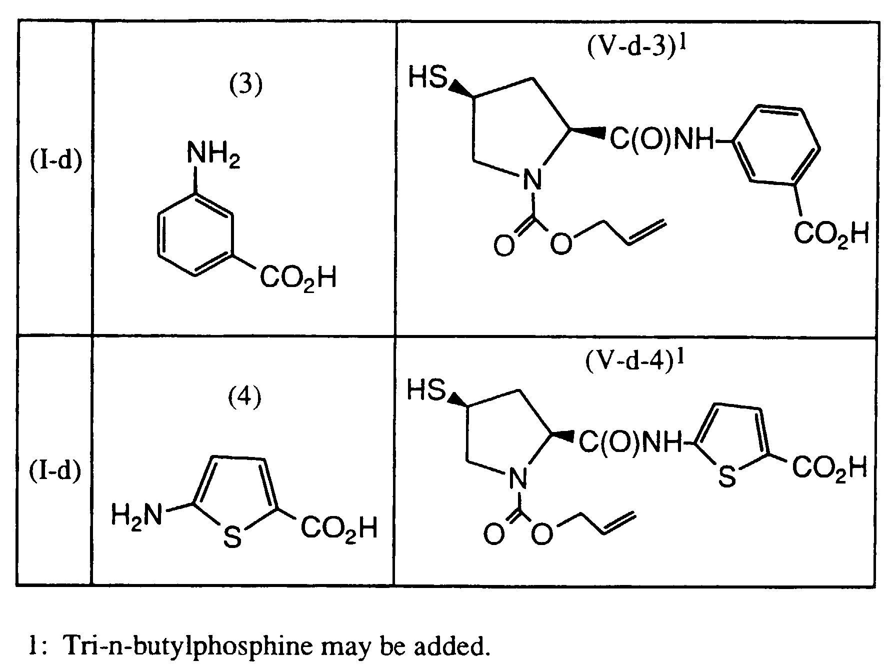

While certain preferred embodiments have been described herein in detail, numerous alternative embodiments are contemplated as falling within the scope of the invention.

## Claims

1. A process for synthesizing a compound of the formula I: 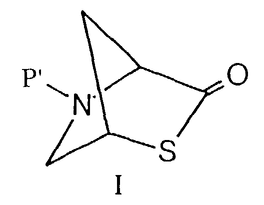 wherein P' is a protecting group selected from t-butoxycarbonyl, p-nitrobenzyloxycarbonyl, benzyloxycarbonyl and allyloxycarbonyl;
comprising
(a) reacting a compound of the formula II: 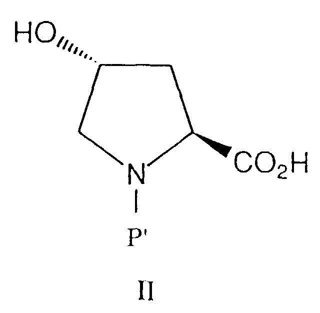 wherein P'is as previously defined with diphenylphosphinic chloride to produce a compound of the formula III: 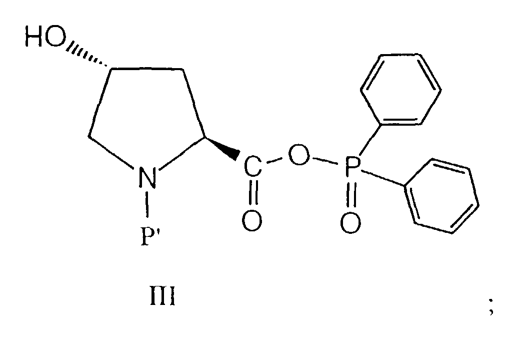
(b) reacting compound Ill with methanesulfonyl chloride to produce a compound of formula IV: 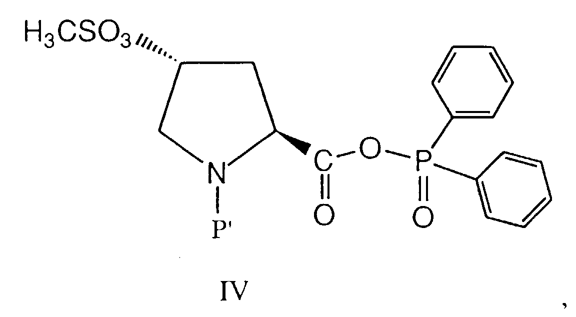 and
(c) combining compound IV with Na₂S in water to produce a compound of formula I.

2. A process of producing a compound of the formula V: 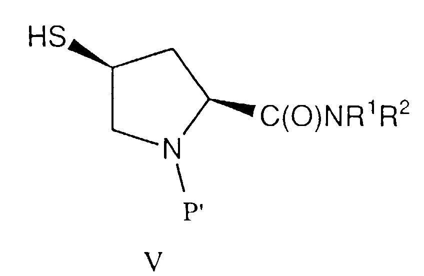 wherein P' is a protecting group selected from t-butoxycarbonyl, p-nitrobenzyloxycarbonyl, benzyloxycarbonyl and allyloxycarbonyl;
R¹ and R² are independently selected from hydrogen, aryl and heteroaryl, where aryl refers to 6-10 membered mono- and bicyclic ring systems, and heteroaryl refers to aromatic 5-10 membered mono- and bicyclic ring systems containing from 1-4 heteroatoms selected from O, S or N, said aryl and heteroaryl groups being unsubstituted or substituted with from 1-3 groups selected from the group consisting of: C₁₋₄ alkyl, C₁₋₄ alkoxy, C₁₋₄ alkylthio, halo, hydroxy, CO₂H, CO₂C₁₋₄ alkyl, NH₂, NHC₁₋₄ alkyl, N(C₁₋₄ alkyl)₂, SO₃H, CN, SO₂NH₂, SO₂C₁₋₄ alkyl, aryl and heteroaryl;
comprising:
(a) reacting a compound of the formula II: 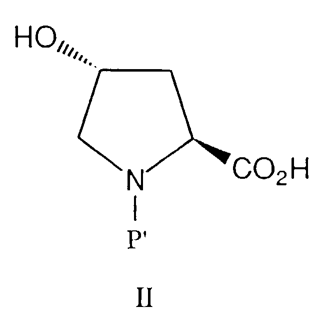 wherein P' is as previously defined with diphenylphosphinic chloride to produce a compound of the formula III: 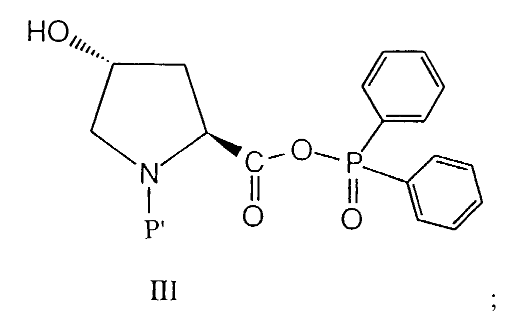
(b) reacting compound III with methanesulfonyl chloride to produce a compound of formula IV: 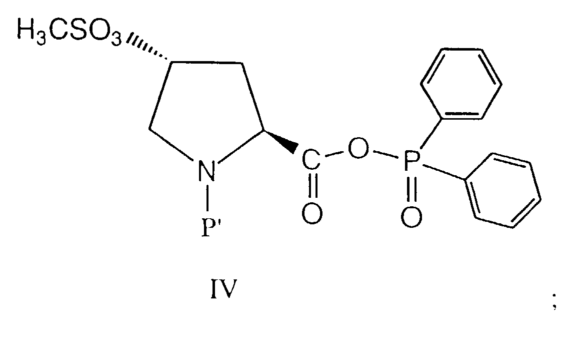
(c) combining compound IV with Na2S in water to produce a compound of formula I: 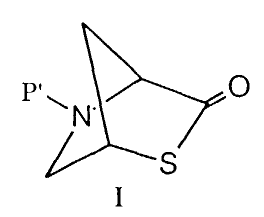
and (d) reacting compound I with NHR¹R² wherein R¹ and R² are as previously defined to produce a compound of formula V.

3. A process in accordance with claim 1 wherein compound II is reacted with diphenylphosphinic chloride in the presence of a base.

4. A process in accordance with claim 3 wherein the base is a trialkylamine.

5. A process in accordance with claim 4 wherein the trialkylamine is selected from the group consisting of diisopropylethylamine and triethylamine.

6. A process in accordance with claim 1 wherein compound III is reacted with methanesulfonyl chloride to produce a compound of formula IV in the presence of a base.

7. A process in accordance with claim 6 wherein the base is selected from the group consisting of pyridine, collidine and lutidine.

8. A process in accordance with claim 1 wherein Na₂S in water is reacted with compound IV to produce a compound of formula 1 at a temperature of about -10°C to about room temperature.

9. A process in accordance with claim 1 wherein P' represents t-butoxycarbonyl or p-nitrobenzyloxycarbonyl.

10. A process in accordance with claim 2 wherein compound II is reacted with diphenylphosphinic chloride in the presence of a base.

11. A process in accordance with claim 10 wherein the base is a trialkylamine.

12. A process in accordance with claim 11 wherein the trialkylamine is selected from the group consisting of diisopropylethylamine and triethylamine.

13. A process in accordance with claim 2 wherein compound III is reacted with methanesulfonyl chloride to produce a compound of formula IV in the presence of a base.

14. A process in accordance with claim 13 wherein the base is selected from the group consisting of pyridine, collidine and lutidine.

15. A process in accordance with claim 1 wherein the Na₂S in water is reacted with compound IV to produce a compound of formula I at a temperature of about -10°C to about room temperature.

16. A process in accordance with claim 2 wherein P' represents t-butoxycarbonyl or p-nitrobenzyloxycarbonyl.

17. A process in accordance with claim 2 wherein NHR¹R² is selected from the group consisting of:

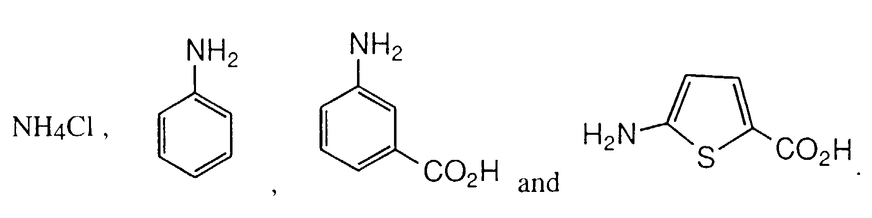

## Patentansprüche

1. Ein Verfahren zur Synthese einer Verbindung der Formel I: 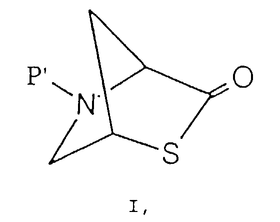 worin P' eine Schutzgruppe ist, ausgewählt aus t-Butoxycarbonyl, p-Nitrobenzyloxycarbonyl, Benzyloxycarbonyl und Allyloxycarbonyl,
umfassend
(a) die Umsetzung einer Verbindung der Formel II: 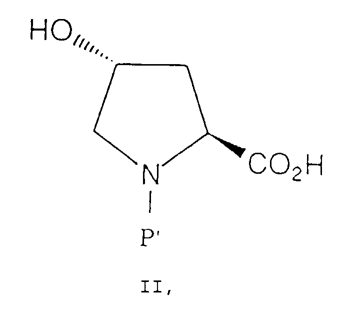 worin P' wie zuvor definiert ist, mit Diphenylphosphinsäurechlorid, um eine Verbindung der Formel III zu erzeugen: 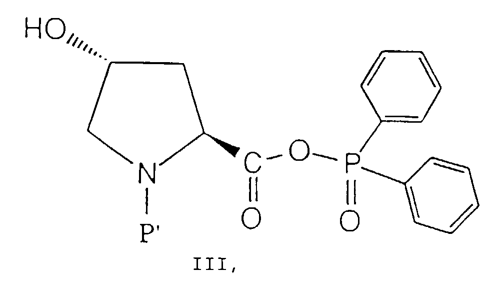
(b) die Umsetzung von Verbindung III mit Methansulfonylchlorid, um eine Verbindung der Formel IV zu erzeugen: 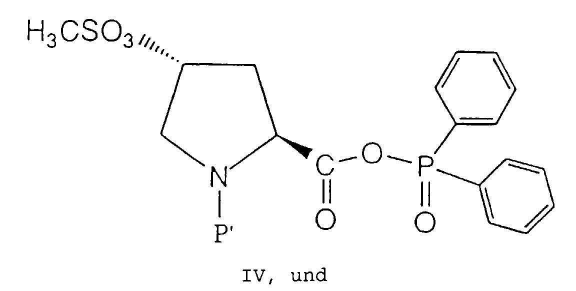
(c) das Vereinen von Verbindung IV mit Na₂S in Wasser, um eine Verbindung der Formel I zu erzeugen.

2. Ein Verfahren zur Herstellung einer Verbindung der Formel V: 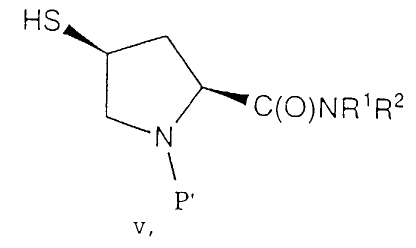 worin P' eine Schutzgruppe ist, ausgewählt aus t-Butoxycarbonyl, p-Nitrobenzyloxycarbonyl, Benzyloxycarbonyl und Allyloxycarbonyl,
R¹ und R² unabhängig ausgewählt sind aus Wasserstoff, Aryl und Heteroaryl, wobei Aryl 6- bis 10gliedrige mono- und bicyclische Ringsysteme bedeutet, und Heteroaryl aromatische 5- bis 10gliedrige mono- und bicyclische Ringsysteme bedeutet, welche 1-4 Heteroatome, ausgewählt aus O, S oder N, enthalten, wobei die Aryl- und Heteroarylgruppen unsubstituiert oder substituiert sind mit 1-3 Gruppen, ausgewählt aus der Gruppe, bestehend aus: C₁₋₄-Alkyl, C₁₋₄-Alkoxy, C₁₋₄-Alkylthio, Halogen, Hydroxy, CO₂H, CO₂C₁₋₄-Alkyl, NH₂, NHC₁₋₄-Alkyl, N(C₁₋₄-Alkyl)₂, SO₃H, CN, SO₂NH₂, SO₂C₁₋₄-Alkyl, Aryl und Heteroaryl,
umfassend:
(a) die Umsetzung einer Verbindung der Formel II: 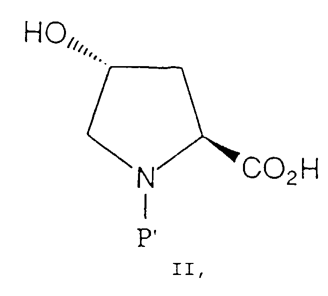 worin P' wie zuvor definiert ist, mit Diphenylphosphinsäurechlorid, um eine Verbindung der Formel III zu erzeugen: 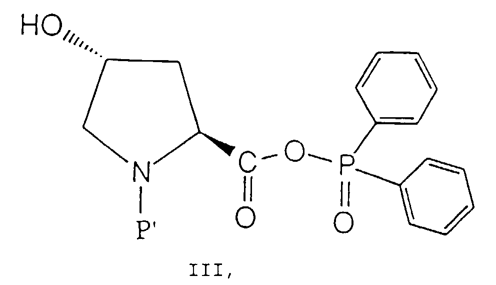
(b) die Umsetzung von Verbindung III mit Methansulfonylchlorid, um eine Verbindung der Formel IV zu erzeugen: 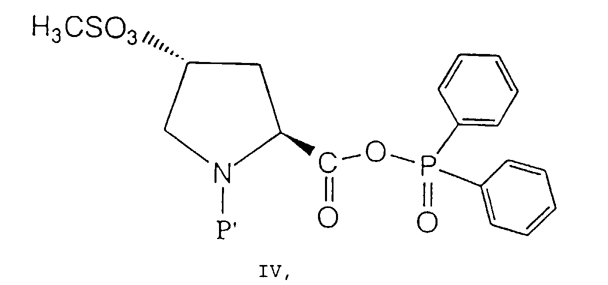
(c) das Vereinen von Verbindung IV mit Na₂S in Wasser, um eine Verbindung der Formel I zu erzeugen: 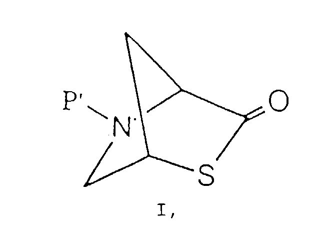
und (d) die Umsetzung von Verbindung I mit NHR¹R², wobei R¹ und R² wie zuvor definiert sind, um eine Verbindung der Formel V zu erzeugen.

3. Ein Verfahren gemäß Anspruch 1, bei dem Verbindung II mit Diphenylphosphinsäurechlorid in Gegenwart einer Base umgesetzt wird.

4. Ein Verfahren gemäß Anspruch 3, bei dem die Base ein Trialkylamin ist.

5. Ein Verfahren gemäß Anspruch 4, bei dem das Trialkylamin ausgewählt ist aus der Gruppe, bestehend aus Diisopropylethylamin und Triethylamin.

6. Ein Verfahren gemäß Anspruch 1, bei dem Verbindung III mit Methansulfonylchlorid in Gegenwart einer Base umgesetzt wird, um eine Verbindung der Formel IV zu erzeugen.

7. Ein Verfahren gemäß Anspruch 6, bei dem die Base ausgewählt ist aus der Gruppe, bestehend aus Pyridin, Collidin und Lutidin.

8. Ein Verfahren gemäß Anspruch 1, bei dem Na₂S in Wasser mit Verbindung IV bei einer Temperatur von etwa -10°C bis etwa Raumtemperatur umgesetzt wird, um eine Verbindung der Formel I zu erzeugen.

9. Ein Verfahren gemäß Anspruch 1, bei dem P' t-Butoxycarbonyl oder p-Nitrobenzyloxycarbonyl bedeutet.

10. Ein Verfahren gemäß Anspruch 2, bei dem Verbindung II mit Diphenylphosphinsäurechlorid in Gegenwart einer Base umgesetzt wird.

11. Ein Verfahren gemäß Anspruch 10, bei dem die Base ein Trialkylamin ist.

12. Ein Verfahren gemäß Anspruch 11, bei dem das Trialkylamin ausgewählt ist aus der Gruppe, bestehend aus Diisopropylethylamin und Triethylamin.

13. Ein Verfahren gemäß Anspruch 2, bei dem Verbindung III mit Methansulfonylchlorid in Gegenwart einer Base umgesetzt wird, um eine Verbindung der Formel IV zu erzeugen.

14. Ein Verfahren gemäß Anspruch 13, bei dem die Base ausgewählt ist aus der Gruppe, bestehend aus Pyridin, Collidin und Lutidin.

15. Ein Verfahren gemäß Anspruch 1, bei dem das Na₂S in Wasser mit Verbindung IV bei einer Temperatur von etwa -10°C bis etwa Raumtemperatur umgesetzt wird, um eine Verbindung der Formel I zu erzeugen.

16. Ein Verfahren gemäß Anspruch 2, bei dem P' t-Butoxycarbonyl oder p-Nitrobenzyloxycarbonyl bedeutet.

17. Ein verfahren gemäß Anspruch 2, bei dem NHR¹R² ausgewählt ist aus der Gruppe, bestehend aus:

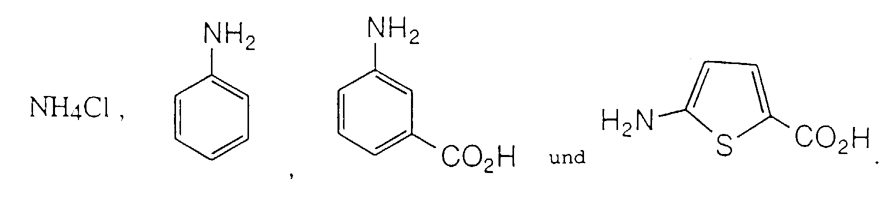

## Revendications

1. Procédé de synthèse d'un composé de formule I: 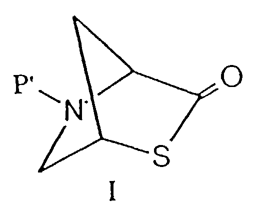 dans laquelle P' est un groupe protecteur choisi parmi les groupes t-butoxycarbonyle, p-nitrobenzyloxycarbonyle, benzyloxycarbonyle et allyloxycarbonyle,
comprenant les étapes consistant à:
(a) faire réagir un composé de formule II: 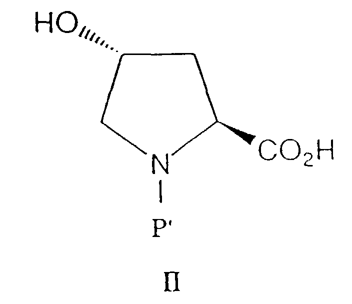 dans laquelle P' est tel que défini ci-dessus, avec du chlorure diphénylphosphinique, pour produire un composé de formule III: 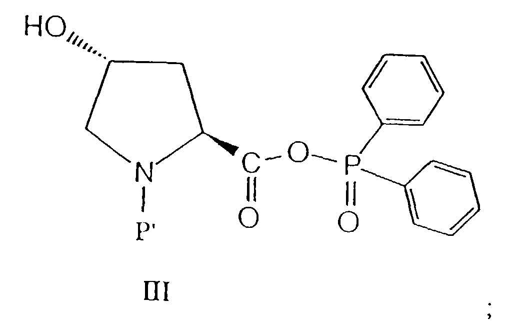
(b) faire réagir le composé III avec du chlorure de méthanesulfonyle pour produire un composé de formule IV: 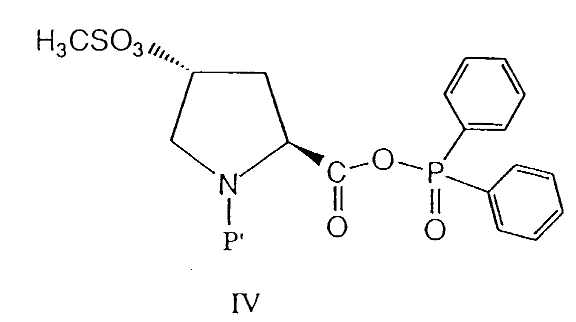 et
(c) combiner le composé IV avec Na₂S dans de l'eau pour produire un composé de formule I.

2. Procédé de production d'un composé de formule V: 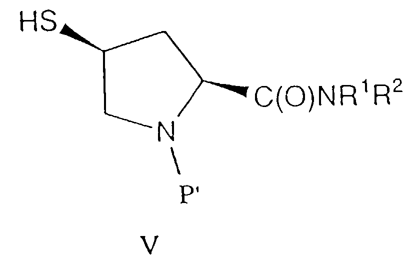 dans laquelle P' est un groupe protecteur choisi parmi les groupes t-butoxycarbonyle, p-nitrobenzyloxycarbonyle, benzyloxycarbonyle et allyloxycarbonyle;
R¹ et R² sont choisis indépendamment parmi des atomes d'hydrogène et des groupes aryle et hétéroaryle, lesdits groupes aryle désignant des systèmes à noyau monocyclique ou bicyclique de 6 à 10 chaînons, et lesdits groupes hétéroaryle désignant des systèmes aromatiques à noyau monocyclique ou bicyclique de 5 à 10 chaînons contenant de 1 à 4 hétéroatomes choisis parmi O, S ou N, lesdits groupes aryle et hétéroaryle étant non substitués ou substitués par 1 à 3 groupes choisis dans le groupe constitué par : les groupes alkyle en C₁-C₄, alcoxy en C₁-C₄, alkylthio en C₁-C₄, halogéno, hydroxy, CO₂H, CO₂-alkyle en C₁-C₄, NH₂, NH-alkyle en C₁-C₄, N(alkyle en C₁-C₄)₂, SO₃H, CN, SO₂NH₂, SO₂-alkyle en C₁-C₄, aryle et hétéroaryle;
comprenant les étapes consistant à:
(a) faire réagir un composé de formule II: 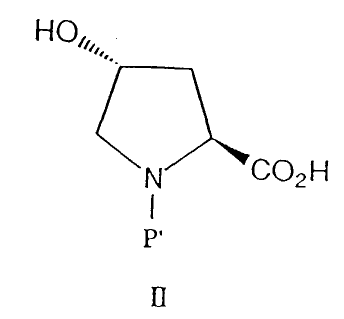 dans laquelle P' est tel que défini ci-dessus, avec du chlorure diphénylphosphinique, pour produire un composé de formule III: 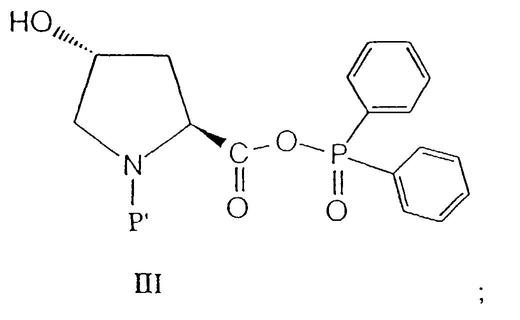
(b) faire réagir le composé III avec du chlorure de méthanesulfonyle pour produire un composé de formule IV: 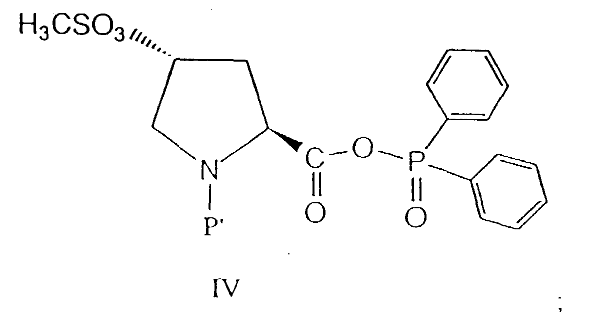
(c) combiner le composé IV avec Na₂S dans de l'eau pour produire un composé de formule I: 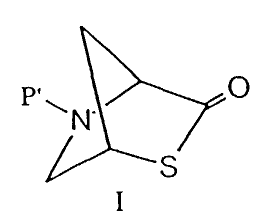 et
(d) faire réagir le composé I avec NHR¹R², où R¹ et R² sont tels que définis ci-dessus, pour produire un composé de formule V.

3. Procédé selon la revendication 1, dans lequel on fait réagir le composé II avec du chlorure diphénylphosphinique en présence d'une base.

4. Procédé selon la revendication 3, dans lequel la base est la trialkylamine.

5. Procédé selon la revendication 4, dans lequel la trialkylamine est choisie dans le groupe constitué par la diisopropyléthylamine et la triéthylamine.

6. Procédé selon la revendication 1, dans lequel le composé III est mis à réagir avec du chlorure de méthanesulfonyle pour produire un composé de formule IV en présence d'une base.

7. Procédé selon la revendication 6, dans lequel la base est choisie dans le groupe constitué par la pyridine, la collidine et la lutidine.

8. Procédé selon la revendication 1, dans lequel on fait réagir Na2S dans de l'eau avec le composé IV pour produire un composé de formule 1 à une température allant d'environ -10°C aux environs de la température ambiante.

9. Procédé selon la revendication 1, dans lequel P' représente le groupe t-butoxycarbonyle ou p-nitrobenzyloxycarbonyle.

10. Procédé selon la revendication 2, dans lequel le composé II est mis à réagir avec du chlorure diphénylphosphinique en présence d'une base.

11. Procédé selon la revendication 10, dans lequel la base est une trialkylamine.

12. Procédé selon la revendication 11, dans lequel la trialkylamine est choisie dans le groupe constitué par la diisopropyléthylamine et la triéthylamine.

13. Procédé selon la revendication 2, dans lequel le composé III est mis à réagir avec du chlorure de méthanesulfonyle pour produire un composé de formule IV en présence d'une base.

14. Procédé selon la revendication 13, dans lequel la base est choisie dans le groupe constitué par la pyridine, la collidine et la lutidine.

15. Procédé selon la revendication 1, dans lequel on fait réagir Na₂S dans de l'eau avec le composé IV pour produire un composé de formule 1 à une température allant d'environ -10°C aux environs de la température ambiante.

16. Procédé selon la revendication 2, dans lequel P' représente le groupe t-butoxycarbonyle ou p-nitrobenzyloxycarbonyle.

17. Procédé selon la revendication 2, dans lequel NHR¹R² est choisi dans le groupe constitué par:
